# EUROPÄISCHE PATENTSCHRIFT

(11) **EP 0 703 962 B1**
(45) Veröffentlichungstag und Bekanntmachung des Hinweises auf die Patenterteilung: **12.03.1997**
(21) Anmeldenummer: 94918844.5
(22) Anmeldetag: 07.06.1994
(51) Int. Cl.: C11D 1/825, A61K 7/50

(54) **ULTRAMILDE TENSIDMISCHUNGEN**
ULTRAMILD SURFACTANT MIXTURES
MELANGES TENSIOACTIFS ULTRADOUX

(30) Priorität: 16.06.1993 DE 4319700
(43) Veröffentlichungstag der Anmeldung: 03.04.1996
(73) Patentinhaber: Henkel Kommanditgesellschaft auf Aktien, 40191 Düsseldorf (DE)
(72) Erfinder: GIESEN, Brigitte, D-40235 Düsseldorf (DE); PITTERMANN, Wolfgang, D-40625 Düsseldorf (DE); SCHMID, Karl, D-40822 Mettmann (DE); STERZEL, Walter, D-40597 Düsseldorf (DE)
(86) Internationale Anmeldenummer: EP9401842
(87) Internationale Veröffentlichungsnummer: WO9429416

(56) Entgegenhaltungen:
- EP-A- 0 384 983
- EP-A- 0 444 262

## Beschreibung

### Gebiet der Erfindung

Die Erfindung betrifft Tensidgemische mit verbesserter dermatologischer Verträglichkeit und vorteilhaftem Kälteverhalten enthaltend Alkyloligoglucoside einer ausgewählten Kettenlängenzusammensetzung.

### Stand der Technik

Alkyloligoglykoside, insbesondere Alkyloligoglucoside stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1, (Okt.1992),** D.Balzer und N.Ripke in **Seifen-Öle-Fette-Wachse 118, 894 (1992)** und B.Brancq in **Seifen-Öle-Fette-Wachse 118, 905 (1992)** genannt werden sollen.

Obschon Alkyloligoglucoside ausgesprochen hautmild sind, besteht dennoch ein ständig steigendes Bedürfnis nach Stoffen mit weiter verbesserter dermatologischer Verträglichkeit. In der Vergangenheit hat man beispielsweise versucht, die hautkosmetische Verträglichkeit von Alkyloligoglucosiden durch Zugabe von amphoteren Tensiden zu verbessern.

Ein Ansatzpunkt zur Herstellung besonders leistungsstarker Alkyloligoglucoside besteht in der Abmischung von Species unterschiedlicher Kettenlänge. So wird beispielsweise in WO 93/07249 (Henkel Corp.) vorgeschlagen, zwei Alkyloligoglucoside der Kettenlänge C₈-C₁₀ und C₁₂-C₁₆ im Verhältnis 50 : 50 bis 90 : 10 Gewichtsteile abzumischen. Die Lehre der Anmeldung geht jedoch dahin, ein Mischungsverhältnis von 60 : 40 bis 80 : 20, also die kurzkettigen Species im Überschuß einzusetzen.

In der Deutschen Offenlegungsschrift **DE-A1 40 05 958** (Hüls) wird ein flüssiges, schäumendes Reinigungsmittel offenbart, das 3 bis 40 Gew.-% eines C₇-C₁₀- und 3 bis 40 Gew.-% C₁₁-C₁₈-Alkyloligoglucosids (ad 100 Gew.-% Wasser) enthalten kann. Es wird vorgeschlagen, die kürzer- und längerkettigen Species im Gewichtsverhältnis 10 : 90 bis 50 : 50, vorzugsweise im Verhältnis 17 : 83 bis 33 : 67 einzusetzen. Ein Hinweis auf besondere Vorteile hinsichtlich der dermatologischen Verträglichkeit der Mischung enthält die Anmeldung nicht.

In der Vergangenheit sind Alkyloligoglucoside unterschiedlicher Kettenlänge vorwiegend im Hinblick auf optimale anwendungstechnische Eigenschaften abgemischt worden. Die nach der Lehre des Stands der Technik erhaltenen Gemische mögen zwar beispielsweise hinsichtlich ihres Schaum- und Reinigungsvermögens zufriedenstellend sein, ihre dermatologische Verträglichkeit sowie ihr Kälteverhalten ist jedoch nicht optimal.

Die Aufgabe der Erfindung hat nun darin bestanden, neue Mischungen von Alkyloligoglucosiden unterschiedlicher Kettenlänge zur Verfügung zu stellen, die frei von den geschilderten Nachteilen sind.

### Beschreibung der Erfindung

Gegenstand der Erfindung sind ultramilde Tensidgemische, enthaltend
a) 48 bis 52 Gew.-% eines Alkyloligoglucosids der Formel (I),

   R¹-O-[G]ₚ (I)

   in der R¹ für Alkylreste mit im wesentlichen 8 bis 10 Kohlenstoffatomen, G für einen Glucoserest und p für Zahlen von 1,3 bis 1,6 steht und
b) 48 bis 52 Gew.-% eines Alkyloligoglucosids der Formel (II),

   R²-O-[G]ₚ (II)

   in der R² für Alkylreste mit im wesentlichen 12 bis 16 Kohlenstoffatomen, G für einen Glucoserest und p für Zahlen von 1,38 bis 1,53 steht.

Überraschenderweise wurde gefunden, daß innerhalb eines sehr engen Mischungsbereiches von Alkyloligoglucosiden unterschiedlicher Kettenlänge Produkte mit besonders hoher dermatologischer Verträglichkeit und Kältestabilität erhalten werden können. Auch wenn ähnliche Mischungen kurzkettiger und langkettiger Alkyloligoglucoside im Verhältnis 50 : 50 als untere Grenzen in de DE-A1 40 05 958 (Hüls) genannt sind, ist die getroffene Auswahl neu und erfinderisch, da weder die Mischungen als solche, noch der mit ihnen verbundene überraschende Effekt vorbeschrieben ist und die Lehre der genannten Schrift in die Richtung von Mischungsverhältnissen weist, bei denen die vorteilhafte dermatologische Verträglichkeit und das verbesserte Kälteverhalten nicht mehr vorliegen.

### Alkyloligoglucoside

Alkyloligoglucoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Indexzahl p in den allgemeinen Formeln (I) und (II) gibt den Oligomerisierungsgrad (DP-Grad), d. h. die Verteilung von Mono- und Oligoglucosiden an und steht für eine Zahl zwischen 1,3 und 1,6 im Formel (I) und zwischen 1,38 und 1,53 im Formel (II) Während p in einer gegebenen Verbindung stets ganzzahlig sein muß, ist der Wert p für ein bestimmtes Alkyloligoglucosid eine analytisch ermittelte rechnerische Größe, die meistens eine gebrochene Zahl darstellt.

Der Alkylrest R¹ kann sich von primären Alkoholen mit im wesentlichen 8 bis 10 Kohlenstoffatomen ableiten. Typische Beispiele sind Caprylalkohol, 2-Ethylhexylalkohol und Caprinalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside (DP = 1,3 bis 1,6), die als Vorlauf bei der destillativen Auftrennung von technischem C₈-C₁₈-Kokosfettalkohol anfallen und Alkylreste mit im wesentlichen 8 bis 10 Kohlenstoffatomen aufweisen. Besonders bevorzugt sind Alkyloligoglucoside der Formel (I), die folgende C-Kettenverteilung im Alkylrest aufweisen:
- C₆ :: 0 - 5 Gew.-%
- C₈ :: 40 - 66 Gew.-%
- C₁₀ :: 30 - 59 Gew.-%
- C₁₂:: 0 - 6 Gew.-%

Der Alkylrest R² kann sich von primären Alkoholen mit im wesentlichen 12 bis 16 Kohlenstoffatomen ableiten. Typische Beispiele sind Laurylalkohol, Myristylalkohol und Cetylalkohol sowie deren technische Gemische, die wie oben beschrieben erhalten werden können. Bevorzugt sind Alkyloligoglucoside auf Basis von gehärtetem C_{10/18}-Kokosalkohol (DP = 1,38 bis 1,53), die Alkylreste mit im wesentlichen 12 bis 16 Kohlenstoffatomen aufweisen. Besonders bevorzugt sind Alkyloligoglucoside der Formel (II), die folgende C-Kettenverteilung im Alkylrest aufweisen:
- C₁₀ :: 0 - 3 Gew.-%
- C₁₂ :: 60 - 75 Gew.-%
- C₁₄ :: 21 - 30 Gew.-%
- C₁₆ :: 0 - 12 Gew.-%
- C₁₈ :: 0 - 3 Gew.-%

### Herstellung der Mischungen

Die Abmischung der Alkyloligoglucoside der Formeln (I) und (II) kann in an sich bekannter Weise erfolgen. Es ist beispielsweise möglich, die konzentrierten Pasten bei erhöhter Temperatur von 40°C miteinander zu verrühren und im Verlauf der Konfektionierung zu den Endprodukten auf die Anwendungskonzentration zu verdünnen. In gleicher Weise können jedoch auch verdünnte Lösungen miteinander vermischt werden. Hierbei handelt es sich um einen rein mechanischen Vorgang, eine chemische Reaktion findet nicht statt.

In einer bevorzugten Ausführungsform der Erfindung ist es jedoch auch möglich, die kürzerkettigen den längerkettigeren Alkyloliglucosiden während deren Herstellung, beispielsweise vor der abschließenden Bleiche zuzusetzen. Ferner ist es möglich - unter Berücksichtigung der Reaktivitätsunterschiede - Fettalkohole einer geeigneten Kettenzusammensetzung mit Glucose in an sich bekannter Weise zu acetalisieren und so die Mischungen in-situ herzustellen. In diesen beiden Fällen werden einheitliche Produkte erhalten.

### Gewerbliche Anwendbarkeit

Die erfindungsgemäßen Tensidgemische zeichnen sich durch eine besonders hohe dermatologische Verträglichkeit aus und sind selbst in Form 50 gew.-%iger wäßriger Pasten nicht hautreizend. Gleichzeitig weisen sie eine besonders vorteilhafte Kältestabilität auf.

### Tenside

Die erfindungsgemäßen Tensidgemische können ihrerseits zusammen mit anderen anionischen, nichtionischen, kationischen und/oder amphoteren bzw. zwitterionischen Tensiden eingesetzt werden.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglyceridsulfate, Fettsäureamid(ether)sulfate, Sulfosuccinate, Sulfosuccinamate, Sulfotriglyceride, Amidseifen, Ethercarbonsäuren, Fettsäureisethionate, Sarcosinate, Tauride, Alkyloligoglucosidsulfate, Alkyl(ether)phosphate und pflanzlichen oder tierischen Eiweißhydrolysaten bzw. deren Kondensationsprodukten mit Fettsäuren. Sofern die anionischen Tenside Polyglycoletherketten enthalten, können sie eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Alk(en)yloligoglykoside, Fettsäureglucamide, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen.

Typische Beispiele für **kationische Tenside** sind quartäre Ammoniumverbindungen und quaternierte Difettsäuretrialkanolaminester.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Oberflächenaktive Mittel

Ein weiterer Gegenstand der Erfindung betrifft die Verwendung der erfindungsgemäßen Tensidgemische zur Herstellung von oberflächenaktiven Mitteln, insbesondere Wasch-, Spül- und Reinigungsmitteln sowie Produkten zur Haar- und Körperpflege, in denen sie in Mengen von 1 bis 99, vorzugsweise 5 bis 30 Gew.-% - bezogen auf die Mittel - enthalten sein können. Typische Beispiele hierzu sind :
*** **Pulverförmige Universalwaschmittel,** enthaltend 10 bis 30 Gew.-% - bezogen auf das Mittel - der erfindungsgemäßen Mischung von Alkyloligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Flüssige Universalwaschmittel,** enthaltend 10 bis 70 Gew.-% - bezogen auf das Mittel - der erfindungsgemäßen Mischung von Alkyloligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Flüssige Feinwaschmittel,** enthaltend 10 bis 50 Gew.-% - bezogen auf das Mittel - der erfindungsgemäßen Mischung von Alkyloligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Flüssige Reinigungs- und Desinfektionsmittel**, enthaltend 10 bis 30 Gew.-% - bezogen auf das Mittel - der erfindungsgemäßen Mischung von Alkyloligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Haarshampoos**, enthaltend 10 bis 30 Gew.-% - bezogen auf das Mittel - der erfindungsgemäßen Mischung von Alkyloligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Haarspülungen,** enthaltend 10 bis 30 Gew.-% - bezogen auf das Mittel - der erfindungsgemäßen Mischung von Alkylligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Schaumbäder,** enthaltend 10 bis 30 Gew.-% - bezogen auf das Mittel - der erfindungsgemäßen Mischung von Alkylligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.
*** **Syndetseifen,** enthaltend 10 bis 50 Gew.-% - bezogen auf das Mittel - der erfindungsgemäßen Mischung von Alkylligoglucosiden der Formeln **(I)** und **(II)** sowie anionische, nichtionische, kationische und/oder amphotere bzw. zwitterionische Tenside und gegebenenfalls weitere Hilfs- und Zusatzstoffe.

**Wasch- und Reinigungsmittel** auf Basis der erfindungsgemäßen Tensidgemische können als Hilfs- und Zusatzstoffe beispielsweise Builder, Salze, Bleichmittel, Bleichaktivatoren, optische Aufheller, Vergrauungsinhibitoren, Lösungsvermittler, Entschäumer und Enzyme enthalten.

Übliche **Builder** sind Natriumaluminiumsilicate (Zeolithe), Phosphate, Phosphonate, Ethylendieamintetraessigsäure, Nitrilotriacetat, Citronensäure und/oder Polycarboxylate. Als Salze bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

**Haarshampoos, Haarlotionen** oder **Schaumbäder** auf Basis der erfindungsgemäßen Tensidgemische können als Hilfs- und Zusatzstoffe beispielsweise Emulgatoren, Ölkomponenten, Fette und Wachse, Verdickungsmittel, Überfettungsmittel, biogene Wirkstoffe, Filmbildner, Duftstoffe, Farbstoffe, Perlglanzmittel, Konservierungsmittel und pH-Regulatoren enthalten.

Übliche **Ölkomponenten** sind Substanzen wie Paraffinöl, Pflanzenöle, Fettsäureester, Squalan und 2-Octyldodecanol, während als **Fette und Wachse** beispielsweise Walrat, Bienenwachs, Montanwachs, Paraffin und Cetylstearylalkohol Verwendung finden.

Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinachaft, veröffentlicht im Verlag Chemie, Weinheim, 1984,** zusammengestellt sind. Diese Farbstoffe werden üblicherweise in Konzentrationen von 0,001 bis 0,1 Gew.-%, bezogen auf die gesamte Mischung, eingesetzt.

Die folgenden Beispiele sollen den Gegenstand der Erfindung näher erläutern, ohne ihn darauf einzuschränken.

### Beispiele

### I. Eingesetzte Alkyloligoglucoside

### Komp.I:

C₈-C₁₀-Alkyloligoglucosid
C-Kettenverteilung im Alkylrest: 45 % C₈, 55 % C₁₀
DP-Grad : 1,59
Plantaren^{(R)} APG 225, Fa.Henkel KGaA, Düsseldorf/FRG

### Komp.II:

C₁₂-C₁₆-Kokosalkyloligoglucosid
C-Kettenverteilung im Alkylrest : 68 % C₁₂, 26 % C₁₄, 6 % C₁₆
DP-Grad : 1,38 - 1,53
Plantaren^{(R)} APG 600, Fa.Henkel KGaA, Düsseldorf/FRG

Alle Produkte wurden in Form 50 gew.-%iger wäßriger Pasten eingesetzt.

### II. Anwendungstechnische Beispiele

Die Bestimmung der Hautreizung erfolgte gemäß der OECD-Methode No.404 und der EEC Directive 84/449 EEC, Pt.B.4. Die angegebenen Reizsummenscores wurden aus den nach 24, 48 und 72 Stunden erhaltenen Reizscores gebildet. Dabei wurde der im Vergleichsversuch V1 ermittelte Reizsummenscore für ein 100 %iges C₁₂-C₁₆-Alkyloligoglucosid (DP = 1,38) zu 100 % gesetzt und die in den übrigen Versuchen erhaltenen Reizsummenscores zu diesem ins Verhältnis gesetzt.

Zur Bestimmung des Langzeit-Kälteverhaltens wurden die Produkte über einen Zeitraum von 5 Monaten bei 10°C gelagert. Die erfindungsgemäßen Gemische waren auch nach dieser Zeit flüssig, die meisten der Vergleichsmischungen hingegen durchkristallisiert.

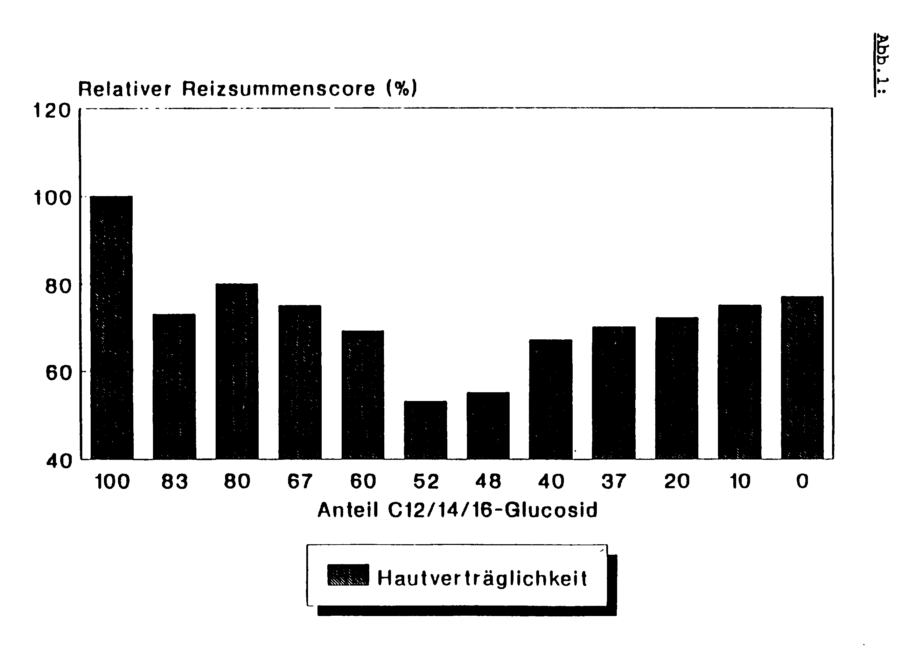

Die Ergebnisse sind in Tab.1 und Abb.1 zusammengefaßt.

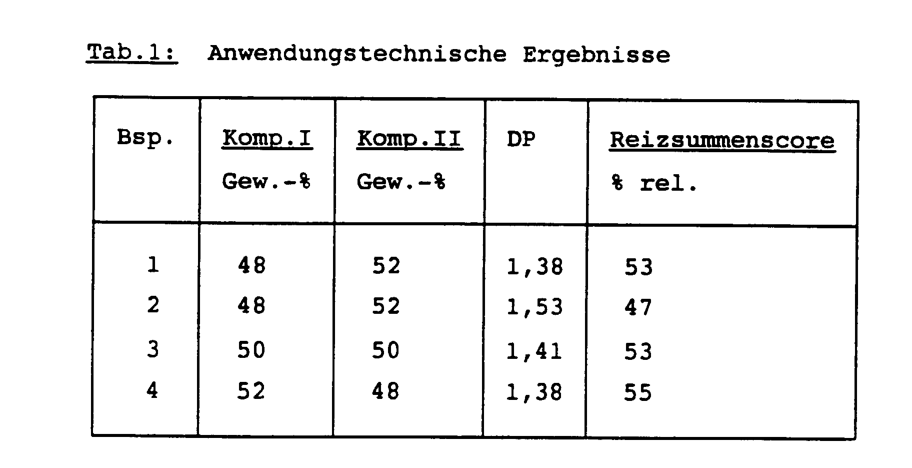

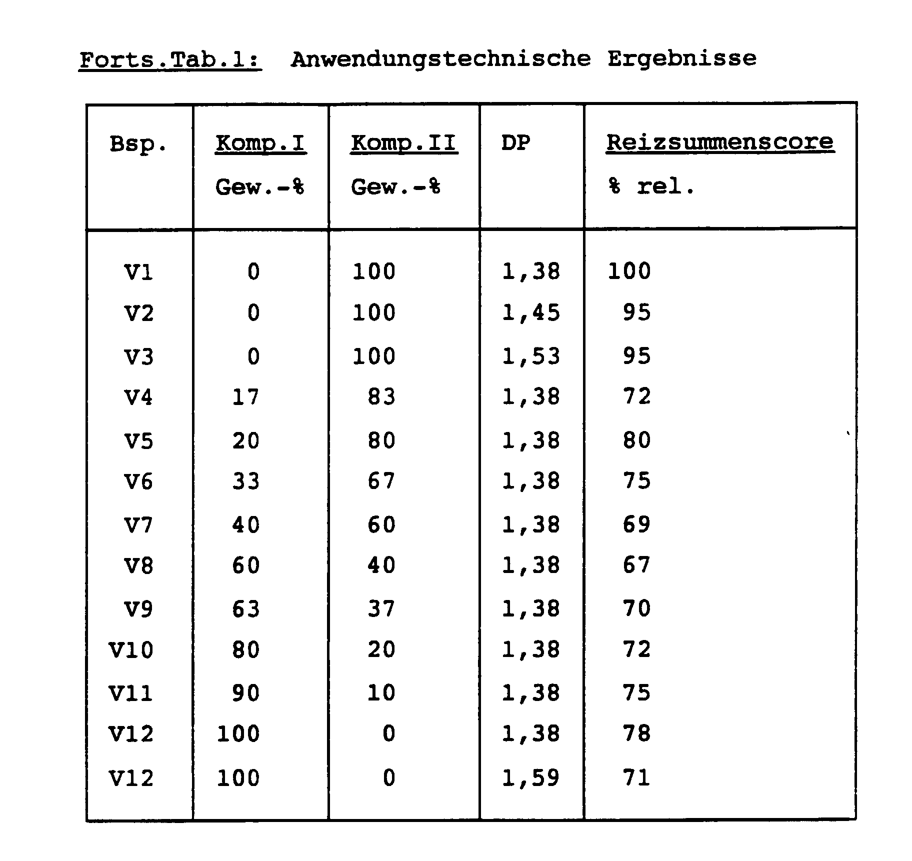

## Patentansprüche

1. Ultramilde Tensidgemische, enthaltend
(a) 48 bis 52 Gew.-% eines Alkyloligoglucosids der Formel **(I)**,
R¹O-[G]ₚ (I)
in der R¹ für Alkylreste mit im wesentlichen 8 bis 10 Kohlenstoffatomen, G für einen Glucoserest und p für Zahlen von 1,3 bis 1,6 steht, und
(b) 48 bis 52 Gew.-% eines Alkyloligoglucosids der Formel **(II)**,
R²O-[G]ₚ (II)
in der R² für Alkylreste mit im wesentlichen 12 bis 16 Kohlenstoffatomen, G für einen Glucoserest und p für Zahlen von 1,38 bis 1,53 steht.

2. Tensidgemische nach Anspruch 1, dadurch gekennzeichnet, daß sie Alkyloligoglucoside der Formel **(I)** enthalten, in der R¹ für Alkylreste der folgenden C-Kettenverteilung steht: C₆ : 0-5 Gew.-%, C₈ : 40-66 Gew.-%, C₁₀ : 30-59 Gew.-% und C₁₂ : 0-6 Gew.-%.

3. Tensidgemische nach den Ansprüchen 1 und 2, dadurch gekennzeichnet, daß sie Alkyloligoglucoside der Formel **(II)** enthalten, in der R² für Alkylreste der folgenden C-Kettenverteilung steht: C₁₀ : 0-3 Gew.-%, C₁₂ : 60-75 Gew.-%, C₁₄ : 21-30 Gew.-%. C₁₆ : 0-12 Gew.-%, C₁₈ : 0-3 Gew.-%.

4. Verwendung von Tensidgemischen nach den Ansprüchen 1 bis 3 zur Herstellung von Wasch-, Spül- und Reinigungsmitteln sowie Produkten zur Haut- und Körperpflege.

## Claims

1. Ultramild surfactant mixtures containing
a) 48 to 52% by weight of an alkyl oligoglucoside corresponding to formula (I):
R¹-O-[G]ₚ (I)
in which R¹ is an alkyl radical essentially containing 8 to 10 carbon atoms, G is a glucose unit and p is a number of 1.3 to 1.6 and
b) 48 to 52% by weight of an alkyl oligoglucoside corresponding to formula (II):
R²-O-[G]ₚ (II)
in which R² is an alkyl radical essentially containing 12 to 16 carbon atoms, G is a glucose unit and p is a number of 1.38 to 1.53.

2. Surfactant mixtures as claimed in claim 1, characterized in that they contain alkyl oligoglucosides corresponding to formula (I), in which R¹ represents alkyl radicals having the following C chain distribution: C₆ : 0 - 5% by weight, C₈ : 40 - 66% by weight, C₁₀: 30 - 59% by weight and C₁₂: 0 - 6% by weight.

3. Surfactant mixtures as claimed in claims 1 and 2, characterized in that they contain alkyl oligoglucosides corresponding to formula (II), in which R² represents alkyl radicals having the following C chain distribution: C₁₀: 0 - 3% by weight, C₁₂: 60 - 75% by weight, C₁₄: 21 - 30% by weight, C₁₆: 0 - 12% by weight, C₁₈: 0 - 3% by weight.

4. The use of the surfactant mixtures claimed in claims 1 to 3 for the production of laundry detergents, dishwashing detergents and cleaning products and skin-care and body-care products.

## Revendications

1. Mélanges de tensioactifs ultra-doux, contenant :
a) 48 à 52 % en poids d'un alkyloligoglucoside de formule (1),
R¹-O-[G]ₚ (I)
où R¹ représente des radicaux alkyles avec essentiellement 8 à 10 atomes de carbone, G est un radical glucose et p représente des nombres de 1,3 à 1,6 et
b) 48 à 52 % en poids d'un alkyloligoglucoside de formule (II),
R²-O-[G]ₚ (II)
où R² représente un radical alkyle avec essentiellement 12 à 16 atomes de carbone, G est un radical glucose et p représente des nombres de 1,38 à 1,53.

2. Mélanges de tensioactifs selon la revendication 1,
caractérisés en ce qu'
ils contiennent des alkyloligoglucosides de formule (I) ou R¹ représente des radicaux alkyles ayant la répartition suivante en chaînes de C : C₆ : 0-5 % en poids, C₈ : 40-66 % en poids, C₁₀ : 30-59 % en poids et C₁₂ : 0-6 % en poids.

3. Mélanges de tensioactifs selon les revendications 1 et 2,
caractérisés en ce qu'
ils contiennent des alkyloligoglucosides de formule (II) où R² représente des radicaux alkyles ayant la répartition suivante en chaînes de C : C₁₀ : 0-3 % en poids, C₁₂ : 60-75 % en poids, C₁₄ : 21-30 % en poids, C₁₆ : 0-12 % en poids, C₁₈ : 0-3 % en poids.

4. Utilisation de mélanges de tensioactifs selon les revendications 1 à 3, pour préparer des produits pour la lessive, la vaisselle et le nettoyage, ainsi que des produits pour les soins du corps et de la peau.
